# EUROPEAN PATENT APPLICATION

(11) **EP 1 876 246 A1**
(43) Date of publication of application: **09.01.2008**
(21) Application number: 07012999.4
(22) Date of filing: 03.07.2007
(51) Int. Cl.: C12Q 1/68

(54) **Self-complementary primers used in LAMP gene amplification method**

(30) Priority: 03.07.2006 JP 2006183299; 29.06.2007 JP 2007172696
(71) Applicant: FUJIFILM Corporation, Minato-ku Tokyo (JP)
(72) Inventor: Iwaki, Yoshihide, Ashigarakami-gun Kanagawa (JP); Mori, Toshihiro, Ashigarakami-gun Kanagawa (JP)
(74) Representative: HOFFMANN EITLE

(57) **Abstract**

A primer for amplifying a target nucleic acid sequence, comprises: a sequence region (a) complementary to a sequence region (a') in the target nucleic acid sequence; and a sequence region (b) having a sequence complementary to a partial sequence of the sequence region (a), in this order from a 3' terminal side to a 5' terminal side of the primer. Use of such primers in LAMP (loop-mediated isothermal amplification) for detecting mutations (e.g. SNPs) or detecting the presence or absence of methylation.

## Description

### Background of the Invention

### 1. Field of the Invention

This invention relates to primers to be used in a novel gene amplification method.

### 2. Description of the Related Art

There is a possibility that a mutation in a gene becomes a cause of a missense mutation which accompanies a change in the translated amino acid, a silent mutation that does not accompany a change of amino acid, or further a frameshift mutation in which the translation frame is shifted due to deletion or insertion of a base. In addition, there is a possibility that a mutation in a gene also results in the abnormal translation of the gene via an abnormal splicing or the like, and there are many cases in which mutations other than the silent mutation accompany a structural or functional change of protein. Furthermore, a mutation in the expression regulation range of a protein has a danger of exerting an influence upon expression regulatory mechanism of the protein.

Among differences on the nucleotide sequence of a nucleic acid, a mutation which is present at a frequency of 1% or more in a certain group is particularly called polymorphisms. In this connection, the term "group" as used herein means a group which is discriminated based on the geographical isolation and subspecies. Among the polymorphisms, polymorphisms due to insertion, deletion or substitution of a single base are particularly called single nucleotide polymorphisms (to be referred to as SNPs hereinafter). SNPs are drawing attention, because they are polymorphisms having most high appearing frequency among human genomes. Since polymorphisms are spreading in a group at a certain frequency, it is considered that they do not accompany changes in the character at all or are controlling not a character which is disadvantageous particularly in terms of the survival or reproduction but a character that can be called constitution in a sense.

Contrary to the polymorphisms, the mutations found at a frequency of less than 1% are mutations which do not spread in a group in the case of human and have a high possibility that most of them are concerned in diseases. That is, the mutations found in hereditary diseases correspond thereto. In addition, even in the case of mutations found in individuals, some of them are concerned in diseases like the case of mutations found in cancers and the like. Detection of such mutations provides decisive information in the diagnosis of corresponding diseases.

Whether or not the nucleotide sequence of a gene is different from a predicted nucleotide sequence can be verified by hybridization of its complementary nucleotide sequence. Illustratively, hybridization of a primer or probe is used. For example, a primer for nucleic acid amplification use can act as the primer only when the target nucleotide sequence has a nucleotide sequence complementary to the primer. Based on this principle, whether or not the target nucleotide sequence is complementary to the primer can be known making use of the nucleic acid amplification product as the index.

PCR (polymerase chain reaction) is known as one of the nucleic acid amplification methods (Science, 230, 1350 - 1354, 1985). However, the method for confirming nucleotide sequence based on PCR has some problems. In the PCR, when a complementary chain is synthesized by mistake, the product becomes a cause of giving a wrong result by functioning as the template of the subsequent reaction. In addition, the PCR has other problems in that a special temperature controlling device is necessary for carrying out the reaction, in that it poses an issue regarding quantitative performance because of exponential progress of the amplification reaction, and in that it is apt to undergo influence of contamination in which a sample or reaction liquid receives pollution from the outside and the mistakenly contaminated nucleic acid functions as the template.

LAMP (loop-mediated isothermal amplification) method is used as a nucleic acid amplification method which can be carried out under isothermal condition and also can maintain the reaction specificity at a high level in comparison with PCR (International Publication 00/28082, T. Notomi et al, , Nucleic Acids Res., 2000, Vol. 28, No. 12, e63) . In addition, a method for detecting SNPs making use of the LAMP method (The 3rd International Workshop on Advanced Genomics 2000, 11, 13 - 14; Yokohama, A Novel SNP Typing Technology Based on the Nucleic Acid Amplification Method, LAMP KANDA, Hidetoshi et *al.*) and a nucleic acid amplification method developed by modifying the LAMP method (International Publication 2002/090538) have also been reported. The LAMP method has a characteristic in that the amplification reaction of nucleic acid is markedly inhibited when the nucleotide sequence of a template is different from the design. Based on this characteristic, the high specificity of the method for detecting mutation based on LAMP method is realized.

### Summary of the Invention

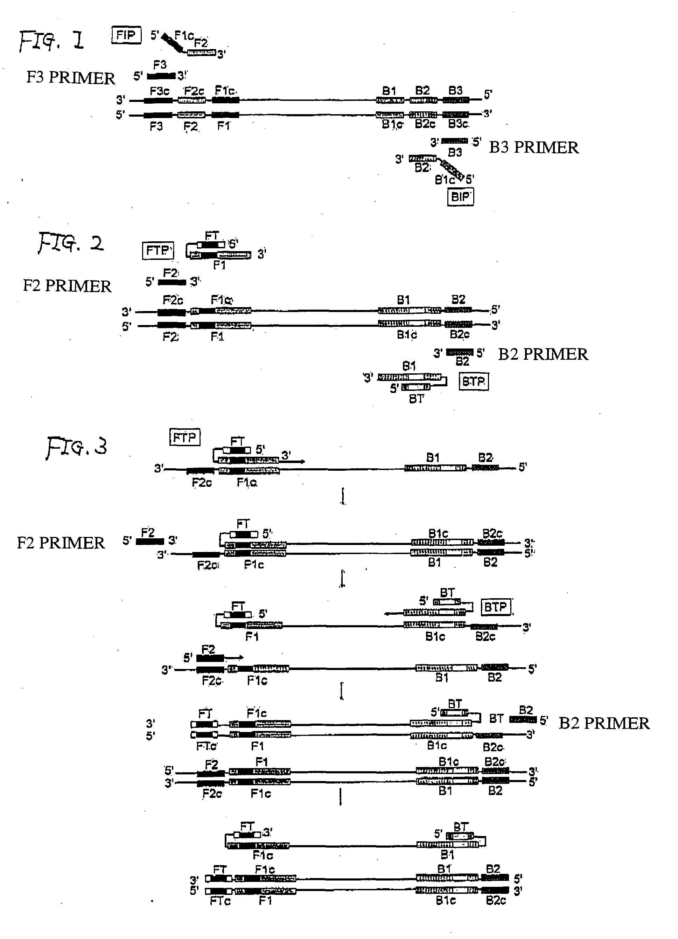

However, the LAMP method and modified methods thereof have a problem in that the degree of freedom for primer designing is limited due to the necessity for a special primer structure. For example, as shown in Fig. 1, it is necessary that the primers to be used in LAMP method have a sequence complementary to the template nucleic acid over two regions. In addition, there are limitations regarding the primer designing, such as the necessity to optimize the distance between primers, melting temperature (to be referred sometimes to as Tm value hereinafter) of each primer, terminal stability of each primer region and GC content thereof, the necessity to avoid formation of extreme secondary structure and the necessity to prevent formation of complementary 3' end (cf. Eiken Chemical Co., Ltd., "LAMP Ho no Genri (The Principle of LAMP method)" (Primer Designing), 2005, internet <URL:http://loopamp.eiken.co.jp/lamp/primer.htm1>).

The present inventors have accomplished the invention by finding that nucleic acid amplification can be carried out by applying a primer having a specified structure to the LAMP method. That is, the invention consists of the following constitution.
(1) A primer for amplifying a target nucleic acid sequence, which comprises:
   a sequence region (a) complementary to a sequence region (a') in the target nucleic acid sequence; and
   a sequence region (b) having a sequence complementary to a partial sequence of the sequence region (a), in this order from a 3' terminal side to a 5' terminal side of the primer.
(2) The primer as described in (1) above,
   wherein each chain length of the sequence regions (a) and (b) is 50 bases or less.
(3) The primer as described in (1) or (2) above,
   wherein a chain length of the sequence complementary to the partial sequence of the sequence region (a) is 10 bases or less.
(4) The primer as described in any of (1) to (3) above, which is utilized in amplifying a target nucleic acid sequence under an isothermal condition.
(5) A method for amplifying a nucleic acid, which comprises:
   carrying out an amplification reaction of a target nucleic acid sequence in a reaction system in which a nucleic acid sample containing the target nucleic acid sequence and the at least one primer as described in any of (1) to (4) above are present.
(6) The nucleic acid amplification method as described in (5) above,
   wherein a primer having a nucleic acid sequence region (c) complementary to a region (c') in the target nucleic acid sequence is further present in the reaction system, with the proviso that the region (c') is present at a further 3' terminal side than the region (a') in the target nucleic acid sequence.
(7) The method as described in (5) or (6) above,
   wherein a mutation recognizing protein is further present in the reaction system.
(8) The method as described in (7) above,
   wherein the mutation recognizing protein is MutS, MSH2 or MHS6, or a mixture of two or more thereof.
(9) The method as described in any of (5) to (8) above,
   wherein a melting temperature adjusting agent is further present in the reaction system.
(10) The method as described in (9) above,
   wherein the melting temperature adjusting agent is dimethyl sulfoxide, betaine, formamide or glycerol, or a mixture of two or more thereof.
(11) The method as described in any of (5) to (10) above,
   wherein the nucleic acid amplification reaction is carried out under an isothermal condition.
(12) A method for detecting presence or absence of a mutation in a target nucleic acid sequence, which comprises the following steps of:
   (1) carrying out an amplification reaction of a target nucleic acid sequence in a nucleic acid sample, in a reaction system in which the nucleic acid sample containing the target nucleic acid sequence and the at least one primer as described in any of (1) to (4) above are present; and
   (2) judging the presence or absence of a mutation in the target nucleic acid sequence based on presence or absence of a product of the nucleic acid amplification reaction.
(13) A method for detecting presence or absence of methylation in a target nucleic acid sequence, which comprises the following steps of:
   (1) carrying out a treatment for replacing a methylated base in a nucleic acid sample containing the target nucleic acid sequence with another base;
   (2) carrying out an amplification reaction of the target nucleic acid sequence using the at least one primer as described in any of (1) to (4) above that comprises a site to be tested for methylation; and
   (3) judging the presence or absence of methylation in the target nucleic acid sequence based on presence or absence of a product of the nucleic acid amplification reaction.
(14) The method as described in (13) above,
   wherein the treatment in the step (1) for replacing a methylated base with another base is a treatment with hydrogen sulfite.
(15) A kit for nucleic acid amplification, which comprises at least:
   the at least one primer as described in any of (1) to (4) above;
   a nucleic acid synthase;
   a substrate; and
   a buffer.
(16) The kit for nucleic acid amplification as described in (15) above, which further comprises a mutation recognizing protein.
(17) The kit for nucleic acid amplification as described in (15) or (16) above, which further comprises a melting temperature adjusting agent.

### Brief Description of the Drawings

Fig. 1 is an illustration showing outlines of the primers to be used in the LAMP method;
Fig. 2 is an illustration showing outlines of the primers to be used in the nucleic acid amplification method of the invention;
Fig. 3 is an illustration showing basic reaction principle of the nucleic acid amplification method of the invention;
Fig. 4 is an illustration showing basic reaction principle of the nucleic acid amplification method of the invention;
Fig. 5 is an illustration showing basic reaction principle of the nucleic acid amplification method of the invention;
Fig. 6 is a figure of a photograph showing the amplified product obtained by an amplification reaction confirmed by a 2% agarose gel (1 x TAE) electrophoresis (Example 1);
Fig. 7 is an illustration showing the amplified product obtained by an amplification reaction confirmed by fluorescence detection (Example 2); and
Fig. 8 is a figure of a photograph showing the amplified product obtained by an amplification reaction, confirmed by a 2% agarose gel (1 x TAE) electrophoresis (Example 2).

### Detailed Description of the Invention

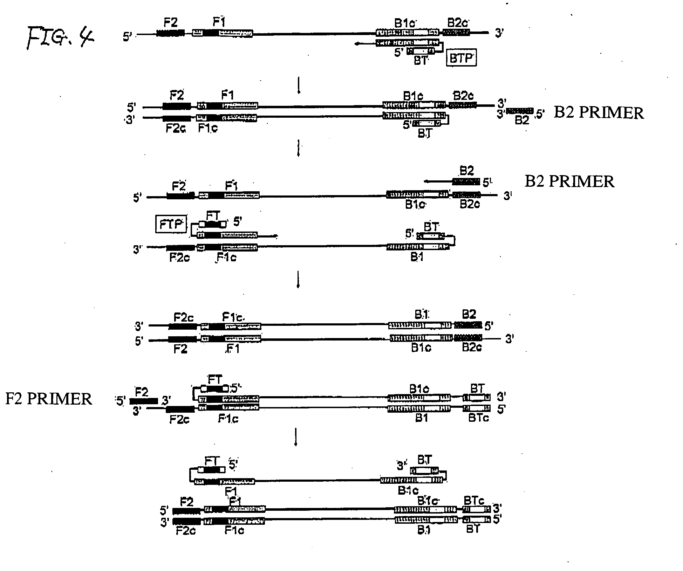

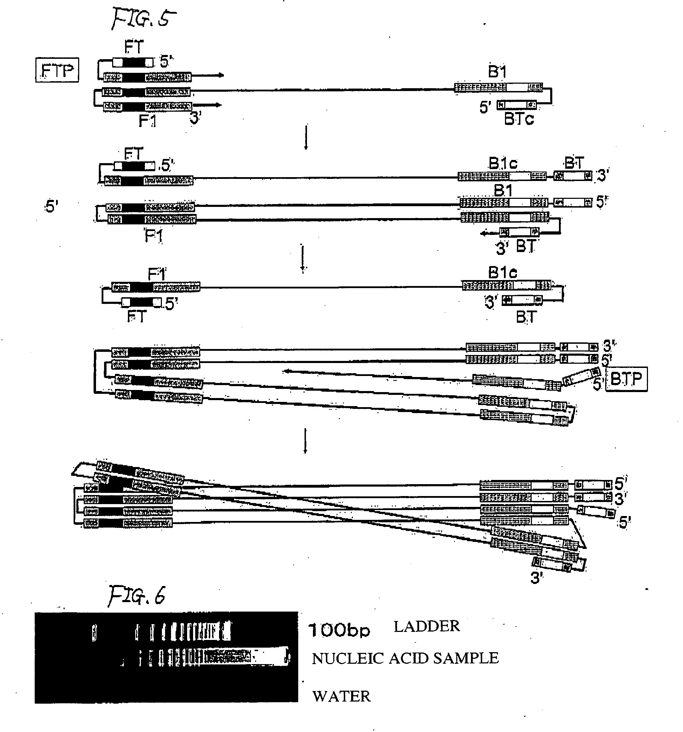

Action mechanism of the nucleic acid amplification reaction when two species of the primer of the invention are used is described based on Figs. 3 and 4 (steps 1 to 5) and Fig. 5 (steps 6 to 9).
Step 1: A nucleic acid sample containing a target nucleic acid sequence is mixed with a reagent and incubated at 60°C. Since the target nucleic acid is in a state of dynamic equilibrium at around 60°C, the primer of the invention (FTP of Fig. 3 or BTP of Fig. 4) hybridizes with a complementary part of the target nucleic acid and elongates therefrom, so that one side of the chain is peeled off to become a single-stranded state.
Step 2: By the action of a strand displacement type nucleic acid synthase, a nucleic acid chain complementary to the template nucleic acid is synthesized, starting at the 3'-end of the F1 region of Fig. 3 (B1 of Fig. 4) of the FTP of Fig. 3 (BTP of Fig. 4). Since this nucleic acid chain has regions self-complementary to the 5'-end side regions (FT and F1 of Fig. 3 and BT and B1 of Fig. 4), it forms a folding region by hybridizing inside the self-molecule.
Step 3: The F2 primer of Fig. 3 (B2 of Fig. 4) hybridizes with the outside of the FTP of Fig. 3 (BTP of Fig. 4), and starting at its 3'-end, the nucleic acid synthesis progresses while peeling off the nucleic acid chain from the previously synthesized FTP of Fig. 3 (BTP of Fig. 4) by the action of the strand displacement type nucleic acid synthase.
Step 4: The nucleic acid chain synthesized from the F2 primer of Fig. 3 (B2 of Fig. 4) and the template nucleic acid become a double-stranded chain. The BTP of Fig. 3 (FTP of Fig. 4) hybridizes with the nucleic acid chain synthesized from the FTP of Fig. 3 (BTP of Fig. 4) which was peeled off and became a single-stranded chain in the step 3, and a complementary nucleic acid synthesis is carried out starting from the 3'-end of this BTP of Fig. 3 (FTP of Fig. 4).
Step 5: The B2 primer of Fig. 3 (F2 of Fig. 4) hybridizes with the outside of the BTP of Fig. 3 (FTP of Fig. 4), and starting at its 3'-end, the nucleic acid synthesis progresses while peeling off the nucleic acid chain from the previously synthesized BTP of Fig. 3 (FTP of Fig. 4) by the action of the strand displacement type nucleic acid synthase. A double-stranded nucleic acid is formed by this step. In addition, since the single-stranded nucleic acid chain synthesized from the BTP of Fig. 3 (FTP of Fig. 4) which was peeled off in the step 5 has self-complementary sequences on both termini, it hybridizes inside the self-molecule and forms a folding region to become a dumbbell type structure (5). This structure becomes the starting structure of amplification cycles of in and after step 6.
Step 6: Steps of in and after the dumbbell structure of Fig. 3 are described based on Fig. 5, wherein the same can be apply to the steps of in and after the dumbbell structure of Fig. 4. In the dumbbell type structure (5) formed in the step 5 (Fig. 3), nucleic acid synthesis progresses starting from the 3' -end FT region using itself as the template, while the 5'-end side folding region is peeled off and elongates. In addition, since the F1c region of the 3' -end side loop is a single-stranded chain, FTP can be hybridized therewith, so that the nucleic acid synthesis progresses starting at the 3'-end of its F1 region, while peeling off the previously synthesized nucleic acid chain from the FT region.
Step 7: Since the nucleic acid chain elongated from the FT region which became a single-stranded chain by being peeled off due to the nucleic acid chain elongated and synthesized from FTP, in the step 6, has a self-complementary region in its 3'-end side, it forms a folding region. Nucleic acid synthesis is started from the 3' -end of the BT region of this folding region using the single-stranded chain itself as the template. Thereafter, this nucleic acid chain is elongated, while peeling off the nucleic acid chain from the FTP forming a double-stranded part, and becomes the structure (7).
Step 8: The nucleic acid chain synthesized from FTP by the process of step 7 becomes a single-stranded chain, and is possessed of self-complementary regions at FT and FTc and BTc and BT, respectively on its both termini, so that it forms a folding region by hybridizing inside the molecule itself. This structure (8) becomes a structure which is complementary to the structure (5) formed by the step 5. In this structure (8), nucleic acid synthesis is carried out using itself as the template, starting at the 3'-end of the BT region similar to the case of the structure (5), and BTP hybridizes with the Blc region which became a single-stranded chain and nucleic acid synthesis is further carried out while peeling off the nucleic acid chain from the BT region. By this, the structure (5) is again formed via the same processes of the steps 5, 6 and 8.
Step 9: In the structure (7), BTP hybridizes with the B1 region which became a single-stranded chain, and a nucleic acid chain is synthesized while peeling off the double-stranded chain part. As a result of these processes, amplification products having such a structure that mutually complementary sequences are repeated on the same chain are synthesized with varied chain lengths. By repeating the above reactions, it becomes possible to synthesize a nucleic acid in a large amount, which is complementary to the target nucleic acid sequence in the template nucleic acid.

The primer of the invention is a primer which renders possible amplification of a target nucleic acid sequence by the aforementioned nucleic acid amplification reactions. That is, the primer of the invention is a primer for amplifying a target nucleic acid sequence, which comprises a sequence region (a) (F1 and B1 of Fig. 2) complementary to a sequence region (a') in the target nucleic acid sequence and a sequence region (b) (FT and BT of Fig. 2) that has a sequence complementary to a partial sequence of the sequence region (a) and forms a folding region, from the 3' terminal side to the 5' terminal tide.

The primer of the invention is composed of deoxynucleotides and/or ribonucleotides and has such a degree of chain length that its base pair bonding with a target nucleic acid can be carried out while keeping necessary specificities under given conditions. Chain length of the primer of the invention is preferably from 10 to 60 bases, more preferably from 20 to 30 bases.

Each chain length of the regions (a) and (b) constituting the primer of the invention is preferably 50 bases or less, more preferably 10 bases or less. The lower limit of each chain length of the regions (a) and (b) is preferably 3 bases or more, more preferably 5 bases or more.

In addition, the sequence region (a) constituting the primer of the invention may have such a structure that it is complementary to a sequence region (a') in the target nucleic acid sequence and can become the starting point of the synthesis of complementary chain of template in the nucleic acid amplification reaction. Also, the sequence region (b) constituting the primer of the invention may has a sequence complementary to a partial sequence of the sequence region (a), and it is desirable that the length of said complementary sequence is such a length that the sequence regions (a) and (b) can form a folding region inside the molecule itself, which is 10 bases or less, more preferably 5 bases or less. The lower limit of the length of said complementary sequence is preferably 3 bases or more, more preferably 5 bases or more.

Also included in the primer of the invention are an oligonucleotide primer composed of unmodified deoxynucleotides and/or modified deoxynucleotides, an oligonucleotide primer composed of unmodified ribonucleotides and/or modified ribonucleotides, a chimeric oligonucleotide primer which comprises unmodified deoxynucleotides and/or modified deoxynucleotides and unmodified ribonucleotides and/or modified ribonucleotides, and the like.

The primer of the invention can be synthesized by any optional method which can be used in the synthesis of oligonucleotides, such as phosphotriester method, H-phosphonate method or thiophosphonate method. The aforementioned first and second primers can be easily obtained when synthesized by the phosphoamidite method using, for example, a DNA Synthesizer Type 394 manufactured by ABI (Applied Biosystems Inc.).

The primer of the invention can be labeled with conventionally known labels. As the labels, digoxin, biotin and the like binding ligands, an enzyme, a fluorescent material, a luminescent material, a radioisotope and the like can be exemplified.

When simply expressed as "5'-end side" or "3'-end side" in this specification, it means the direction in the chain which is regarded as the template in all cases. Also, when described that the 3'-end side becomes the starting point of complementary chain synthesis, it means that the 3'-end side -OH group is the starting point of complementary chain synthesis.

The "oligonucleotide" according to the invention means a polynucleotide having particularly small number of the constituting bases. A polynucleotide having the number of bases of generally from about 2 to about 100, more generally from about 2 to 50, is called oligonucleotide, though not limited to these numerical values.

The "target nucleic acid" or "target nucleic acid sequence" as used in the invention means a nucleotide sequence which constitutes the nucleic acid to be synthesized in the invention. In addition, when amplification of nucleic acid is carried out based on the nucleic acid amplification method of the invention, a nucleotide sequence which constitutes the nucleic acid to be amplified is the target nucleic acid sequence.

In general, a nucleotide sequence of sense chain directing from the 5'-end side to the 3'-end side is described as the nucleotide sequence of nucleic acid. In addition to the nucleotide sequence of sense chain, the target nucleotide sequence according to the invention also includes nucleotide sequence of its complementary chain, namely antisense chain. That is, the aforementioned "target nucleic acid" or "target nucleic acid sequence" is used as a term which means at least either one of the nucleotide sequence to be amplified and its complementary chain.

According to the invention, the target nucleic acid sequence is not limited to the nucleotide sequence of a nucleic acid to be used as the template. Accordingly, the target nucleic acid sequence may consist of the same nucleotide sequence of the template or of a different nucleotide sequence. A mutation can be introduced into the template nucleotide sequence, or a target nucleotide sequence consisting of a nucleotide sequence prepared by connecting a part of the template nucleotide sequence can also be synthesized.

According to the invention, the "template" means a nucleic acid as one side which becomes the template of complementary chain synthesis. The complementary chain having a nucleotide sequence complementary to the template has a meaning as a chain which corresponds to the template, but the relationship between them is only a relative thing to the end. That is, the chain synthesized as a complementary chain can function as the template again.

According to the invention, there are a case in which a nucleotide sequence contained in the template nucleic acid is directly synthesized as the target nucleic acid sequence and a case in which synthesis of a nucleic acid having a nucleotide sequence different from the template nucleic acid is the purpose. As the nucleic acid having a nucleotide sequence different from the template nucleic acid, for example, a case in which a mutation is introduced into the nucleotide sequence contained in the template nucleic acid or in which regions separately presenting on the template nucleic acid are synthesized as a continuing nucleotide sequence can be cited. In addition, the target nucleic acid sequence of the invention can be made into a nucleotide sequence in which nucleotide sequences derived from different nucleic acids are connected to each other.

The "synthesis of nucleic acid" according to the invention means elongation of a nucleic acid from an oligonucleotide used as the synthesis starting point. When formation of other nucleic acid and elongation reaction of the formed nucleic acid occur continuously, in addition to the synthesis, such a series of reactions are called "amplification of nucleic acid" as a whole.

The "hybridize" according to the invention means that a nucleic acid forms a double helix structure by base pair bonding based on the Watson-Crick model. Accordingly, even when the nucleic acid chain constituting the base pair bonding is a single chain, it is hybridization when a complementary nucleotide sequence inside the molecule forms base pair bond. According to the invention, the terms "anneal" and "hybridize" have the same meaning from the viewpoint that nucleic acid forms a double helix structure by base pair bonding.

A nucleotide sequence which is not perfectly complementary is included in the term "complementary" as used in the invention for characterizing the primer-constituting nucleotide sequence. Namely, complementary to a certain sequence means a sequence which can hybridize under a stringent condition and can provide starting point of the complementary chain synthesis.

The aforementioned stringent condition can be determined depending on the Tm value of the double-stranded chain of the primer of the invention with a complementary chain thereof, salt concentration of the hybridization solution and the like, and for example, J. Sambrook, E.F. Frisch and T. Maniatis; Molecular Cloning 2nd edition, Cold Spring Harbor Laboratory (1989) and the like can be used as references.

For example, when hybridization is carried out at a temperature slightly lower than the melting temperature of a primer to be used, the primer can be hybridized specifically with the target nucleic acid. Such a primer can be designed using a commercially available primer construction software such as Primer 3 (mfd. by Whitehead Institute for Biomedical Research). According to a preferred embodiment of the invention, the primer which hybridized with a certain target nucleic acid comprises entire or partial sequence of the nucleic acid molecule complementary to the target nucleic acid.

According to the invention, the "nucleic acid" means DNA, RNA or a chimeric molecule thereof. The "nucleic acid" has the same meaning as the term "polynucleotide". The nucleic acid may be a natural product or an artificially synthesized counterpart. In addition, even in the case of a nucleotide derivative consisting of a partially or entirely artificial structure, it is included in the nucleic acid of the invention with the proviso that it can form base pair bond. Also, the number of bases constituting the nucleic acid of the invention is not particularly limited.

The invention provides a nucleic acid sample containing a target nucleic acid sequence, and a method for amplifying a nucleic acid, which comprises carrying out an amplification reaction of the target nucleic acid sequence in the nucleic acid sample, in a reaction system wherein the primer of the invention is present. It is desirable to use at least one species of the primer of the invention in the nucleic acid amplification reaction of the invention. That is, the primer of the invention may be used in combination with other primer, or two species of the primer of the invention may be used.

Preferred as the other primer to be used in combination with the primer of the invention is a primer which contains a sequence (X') capable of hybridizing with a sequence (X) of a 3'-end moiety of a complementary sequence of the target nucleic acid sequence, in the 3' -end moiety, and also contains a sequence that hybridizes with a complementary sequence of a sequence (Y) presenting in further 5'-end side than the sequence (X) of the complementary sequence of said target nucleic acid sequence, in the 5'-end side of the sequence (X'). Regarding desirable designing standard for such an other primer, it is as described in the foregoing on the primer of the invention.

Regarding the nucleic acid amplification reaction of the invention, it is desirable that a primer having a nucleic acid sequence region (c) complementary to a region (c') in the target nucleic acid sequence [with the proviso that the region (c') is present at a further 3' terminal side than the region (a') in the target nucleic acid sequence] is further present in the reaction system. Said primer may be any primer which can be used in the nucleic acid amplification reaction as the starting point of the synthesis of complementary chain of the template, and can be designed for example in the same manner as the case of primers which are used in PCR and the like conventionally known methods, and its chain length is preferably from 10 to 50 bases, more preferably from 15 to 30 bases.

The nucleic acid sample or template nucleic acid, which contains a target nucleic acid sequence and is used in the nucleic acid amplification reaction of the invention, can be isolated for example from blood, tissues, cells, animals, plants and the like living body-derived samples or microorganisms-derived samples separated from food, soil, waste water and the like. Isolation of nucleic acid samples can be carried out by an optional method such as a lysis treatment with a surfactant, a sonic treatment, shaking agitation using glass beads or a method which uses French press. In addition, when an endogenous nuclease is present, it is desirable to purify the isolated nucleic acid. It is possible to carry out purification of the nucleic acid by, for example, phenol extraction, chromatography, ion exchange, gel electrophoresis, density-dependent centrifugation and the like.

More illustratively, as the aforementioned nucleic acid sample or template nucleic acid, it is possible to use all of the double-stranded nucleic acids such as genomic DNA and PCR fragments isolated by the aforementioned methods and single-stranded nucleic acids such as cDNA prepared from total RNA or mRNA by the reverse transcription reaction. In the case of the aforementioned double-stranded nucleic acid, it can be used most suitably when converted into a single strand by carrying out a denaturation step (denaturing).

The enzyme to be used in the aforementioned reverse transcription reaction is not particularly limited, with the proviso that it has the activity to synthesize cDNA using RNA as the template, and its examples include various reverse transcriptases such as avian myeloblastosis virus-derived reverse transcriptase (AMVRTase), Rous-associated virus 2 reverse transcriptase (RAV-2 RTase) and Moloney mouse leukemia virus-derived reverse transcriptase (MMLV RTase). In addition to these, it is possible also to use a DNA polymerase which is jointly possessed of a reverse transcription activity.

Even when the target nucleic acid is a double-stranded nucleic acid, this can be used as such in the nucleic acid amplification reaction, but annealing of a primer to the template nucleic acid can also be efficiently carried out by converting this into a single strand through its denaturation as occasion demands. Increase of temperature to about 95°C is a desirable nucleic acid denaturation method. Its denaturation through the increase of pH is also possible, but in that case, it is necessary to lower the pH in order to allow the primer to hybridize with the target nucleic acid.

The DNA polymerase to be used in the nucleic acid amplification reaction of the invention may have a chain substitution (strand displacement) activity (strand displacement ability), any one of psychrophilic, mesophilic and thermostable counterparts can be suitably used. Also, this DNA polymerase may be either a natural origin or a mutant prepared by artificially adding a mutation. In addition, it is desirable that this DNA polymerase has substantially no 5'→3' exonuclease activity.

As the DNA polymerase to be used in the nucleic acid amplification reaction of the invention, a 5'→3' exonuclease activity-deleted mutant of DNA polymerase derived from a strain belonging to the genus of thermophilic bacillus such as *Bacillus stearothermophilus* (to be referred to as "*B*. *st*" hereinafter) or *Bacillus caldotenax* (to be referred to as "*B. ca*" hereinafter), a Klenow fragment of *Escherichia coli (E. coli*)-derived DNA polymerase I and the like can be exemplified.

Also, as the DNA polymerase to be used in the nucleic acid amplification reaction of the invention, Vent DNA polymerase, Vent (Exo-) DNA polymerase, DeepVent DNA polymerase, DeepVent (Exo-) DNA polymerase, Φ 29 phage DNA polymerase, MS-2 phage DNA polymerase, Z-Taq DNA polymerase, Pfu DNA polymerase, Pfu turbo DNA polymerase, KOD DNA polymerase, 9° Nm DNA polymerase, Therminater DNA polymerase and the like can be exemplified.

Also, when a DNApolymerase which is jointlypossessed of a reverse transcription activity, such as *B*. *ca* BEST DNA polymerase or *B. ca* (exo-) DNA polymerase, is used in the nucleic acid amplification reaction of the invention, the reverse transcription reaction from total RNA or mRNA and the DNA polymerase reaction using cDNA as the template can be carried out with only one species of polymerase. In addition, the DNA polymerase may also be used in combination with MMLV reverse transcriptase or the like reverse transcriptase.

As the other reagents to be used in the nucleic acid amplification reaction of the invention, magnesium chloride, magnesium acetate, magnesium sulfate or the like catalyst, dNTPmix or the like substrate and Tris-HCl buffer, Tricine buffer, sodium phosphate buffer, potassium phosphate buffer or the like buffer can for example be used. In addition, dimethyl sulfoxide, betaine (N,N,N-trimethylglycine) and the like additive agents and the acidic substances and cationic complexes described in International Publication No. 99/54455 may also be used.

In the nucleic acid amplification reaction of the invention, a mutation recognizing protein may be added to the reaction system. The term "mutation" as used herein means a different base (a base pair in the case of double-stranded nucleic acid) in the target nucleic acid when compared with a control nucleic acid. In addition, the "mutation recognizing protein" is a protein which binds to a mismatch site when such a mismatch is present in a double-stranded nucleic acid or which recognizes a mutation in the double-stranded nucleic acid and binds thereto.

According to the invention, the "mismatch" means that a set of base pair selected from adenine (A), guanine (G), cytosine (C) and thymine (T) (uracil (U) in the case of RNA) is not a normal base pair (a combination of A with T or a combination of G with C). Not only one mismatch but also two or more of continued mismatches, a mismatch formed by the insertion and/or deletion of one or two or more bases and a combination thereof are also included in the mismatch.

When the mutation recognizing protein (also to be called mismatch binding protein or mismatch recognizing protein) is added to the reaction system, the mutation recognizing protein binds to the mutated site of the double-stranded nucleic acid so that the nucleic acid amplification reaction is inhibited. This results in the advantage in that nonspecific nucleic acid amplification reaction is inhibited in the case of the presence of a mutation in the double-stranded nucleic acid.

As the mutation recognizing protein, MutS, MSH2 and MSH6 can for example be suitable cited, and their origins are not limited with the proviso that they can recognize mutation in the double-stranded nucleic acid.

The mutation recognizing protein to be used in the invention may be partial peptides of such these proteins, with the proviso that they can recognize mutation in the double-stranded nucleic acid. In addition to this, the mutation recognizing protein may be a fusion protein with other protein such as glutathione-S-transferase.

In addition, the mutation recognizing protein may be a protein (mutant) consisting of an amino acid sequence in which one or two or more amino acids in its wild type protein are substituted, deleted, added and/or inserted, with the proviso that it can recognize mutation in the double-stranded nucleic acid. Such a mutant is generated sometimes in the natural world, but it is possible also to artificially prepare it optionally making use of a conventionally known method.

It is possible to prepare the mutation recognizing protein as a natural protein or a recombinant protein, by optionally combining anion exchange column, cation exchange column, gel filtration column chromatography, ammonium sulfate fractionation and the like conventionally known methods. In addition, in the case of a recombinant protein having a large expression quantity, it is also possible to prepare it easily only by a chromatography using a cation exchange column and a gel filtration column.

Contact of the double-stranded nucleic acid with a mutation recognizing protein according to the method of the invention is carried out under such conditions that said protein can bind to the mutation site in said double-stranded nucleic acid (e.g., appropriate pH, solvent, ionic environment and temperature). The reaction temperature and salt concentration, kinds of ions, pH of the buffer and the like detailed conditions can be optionally adjusted.

It is known that a mutation recognizing protein sometimes binds also to a single strand, and binding of such a mutation recognizing protein to the single-stranded nucleic acid is inhibited by a single strand binding protein. Accordingly, when a mutation recognizing protein is used in the nucleic acid amplification method of the invention, it is desirable to use a single strand binding protein (SSB) in combination. Said single strand binding protein can be made into optional SSB in this technical field.

In addition, it is known that a mutation recognizing protein sometimes binds to a double-stranded nucleic acid which does not contain a mismatch, but such a wrong binding can be prevented by activating the mutation recognizing protein in advance using an activator. Accordingly, when a mutation recognizing protein is used in the nucleic acid amplification method of the invention, it is desirable to use it by activating in advance by an activator.

The aforementioned activator for activating the mutation recognizing protein can be optionally selected by those skilled in the art and therefore has no particular limitation, but is preferably ATP (adenosine 5'-triphosphate), ADP (adenosine 5'-diphosphate), ATP-γ-S (adenosine 5'-O-(3-thiotriphosphate)), AMP-PNP (adenosine 5'-[β,γ-imido]triphosphate) or the like compound, or one of the nucleotides which can bind to the mutation recognizing protein. Activation of the mutation recognizing protein can be carried out by incubating the mutation recognizing protein and an activator at room temperature for a period of from several seconds to several minutes.

In order to improve amplification efficiency of nucleic acid, a melting temperature adjusting agent can be added to the reaction solution in the nucleic acid amplification reaction of the invention. In general, melting temperature of a nucleic acid is determined by the illustrative nucleotide sequence of the double strand forming part in the nucleic acid. By adding a melting temperature adjusting agent to the reaction solution, this melting temperature can be changed, so that it becomes possible to adjust strength of the double strand formation in the nucleic acid under a certain temperature. A general melting temperature adjusting agent has the effect to lower the melting temperature.

By adding the aforementioned melting temperature adjusting agent, melting temperature of the double strand forming part between two nucleic acids can be lowered, or in other words, it becomes possible to reduce strength of the double strand. Accordingly, when such a melting temperature adjusting agent is added to the reaction solution in the aforementioned nucleic acid amplification reaction, it becomes possible to efficiently convert the double-stranded part into a single strand in the nucleic acid region rich in the GC content which forms a strong double strand and in the region that forms a complex secondary structure, and since this renders possible easy hybridization of the next primer with the intended region after completion of the elongation reaction by the first primer, the nucleic acid amplification efficiency can be improved.

The melting temperature adjusting agent to be used in the invention and its concentration in the reaction solution are properly selected by the person skilled in the art, by taking into consideration other reaction conditions which exert influences upon the hybridization conditions, such as salt concentration and reaction temperature. Accordingly, though not particularly limited, the melting temperature adjusting agent is preferably dimethyl sulfoxide (DMSO), betaine, formamide or glycerol or an optional combination thereof, of which dimethyl sulfoxide (DMSO) is more preferable.

In the nucleic acid amplification reaction of the invention, an enzyme stabilizer can also be added to the reactionsolution. Since enzymes in the reaction solution are stabilized by this, it becomes possible to improve the nucleic acid amplification efficiency. The enzyme stabilizer to be used in the invention may be any agent known in this technical field, such as glycerol, bovine serum albumin or a saccharide, and is not particularly limited.

In addition, in the nucleic acid amplification reaction of the invention, a reagent can also be added to the reaction solution in order to reinforce heat resistance of the DNA polymerase, reverse transcriptase and the like enzymes. Since enzymes in the reaction solution are stabilized by this, it becomes possible to improve the synthesis efficiency and amplification efficiency of nucleic acid. Such a reagent may be any substance known in this technical field and is not particularly limited, but is preferably trehalose, sorbitol, mannitol or a mixture of two or more species thereof.

The nucleic acid amplification reaction of the invention which uses primers can be carried out under isothermal condition. The term "isothermal" as used herein means that the enzymes and primers are kept under an almost constant temperature condition so that they can substantially function. The almost constant temperature condition means that not only the set temperature is accurately maintained but also a slight change in the temperature is acceptable within such a degree that it does not spoil substantial functions of the enzymes and primers. For example, a change in temperature of approximately from 0 to 10°C is acceptable.

The nucleic acid amplification reaction under a constant temperature condition can be carried out by keeping the temperature at such a level that activity of the enzyme to be used can be maintained. In addition, in order to effect annealing of the primer with the target nucleic acid in said nucleic acid amplification reaction, for example, it is desirable to set the reaction temperature to the temperature of around the Tm value of the primer or lower than that, and it is more desirable to set it at a level of stringency by taking the Tm value of the primer into consideration. Thus, this temperature is set to a range of preferably from about 20°C to about 75°C, more preferably from about 35°C to about 65°C. In said nucleic acid amplification reaction, the amplification reaction is repeated until the enzyme is inactivated or one of the reagents including primers is used up.

Since it is possible to carry out the nucleic acid amplification reaction of the invention under isothermal condition as described in the above, a possibility of causing inactivation of the nucleic acid amplification enzymes (DNA polymerase and the like) is low in comparison with the conventional PCR method. Accordingly, the nucleic acid amplification reaction which uses the primer of the invention is also effective for the amplification of target nucleic acids including non-natural nucleotides, wherein a nucleic acid amplification enzyme having no heat resistance is used. In this connection, the term "non-natural nucleotides" means nucleotides which comprise otherbases than the bases contained in the natural nucleotides (adenine, guanine, cytosine and thymine or uracil) and can be incorporated into a space of nucleotide sequence, and their examples include xanthosines, diaminopyridines, isoG, isoC and the like.

The enzyme to be used in the amplification of nucleic acids including non-natural nucleotides are not particularly limited with the proviso that it can amplify such target nucleic acids. In addition, a substance which improves heat resistance of the nucleic acid amplification enzyme, such as trehalose, can also be added to the reaction liquid, because this renders possible efficient amplification of a target nucleic acid sequence comprising a non-natural nucleotide.

In the nucleic acid amplification method of the invention, the nucleic acid amplification reaction may be carried out by preparing the primers of the invention for each of the target nucleic acid sequences, immobilizing these two or more primers onto a solid phase carrier in such a manner that they can be mutually recognized, and using these immobilized primers. This renders possible simultaneous amplification of two or more target nucleic acids and detection of the respective amplification products thereof in a distinguishable manner. Detection of the amplification products can be carried out using an intercalator or the like. For example, by immobilizing two or more primers at respective specified positions onto a flat solid phase carrier in advance, the amplified target nucleic acids can be specified based on the positions where the amplification products were detected after the nucleic acid amplification reaction and detection of the amplification products.

In addition, the presence of the amplification products obtained by the nucleic acid amplification method of the invention can be detected by any other method. When other detection method is carried out using primers immobilized onto a solid phase carrier, the solid phase carrier may be separated from the amplification product as occasion demands. Separation of the solid phase carrier from the amplification product can be carried out by a method conventionally known in this technical field.

As the other detection method, detection of an amplification product by general gel electrophoresis can be exemplified. According to this method, it can be detected, for example, using ethidium bromide, SYBR Green or the like fluorescent material. Regarding still another detection method, it can also be detected by using a marker probe having biotin or the like label and allowing this to hybridize with the amplification product. It is possible to detect biotin based on its binding with fluorescence-labeled avidin or avidin linked to peroxidase or the like enzyme.

Since the nucleic acid synthesized by the nucleic acid amplification reaction of the invention consists of a complementary nucleotide sequence, the majority thereof form base pair bonds. Making use of this characteristic, product of the nucleic acid amplification reaction can be detected. When ethidium bromide, SYBR Green or the like fluorescent pigment as a double strand-specific intercalator is added in advance to the reaction system and then the nucleic acid amplification reaction of the invention is carried out, increase in the fluorescence intensity is observed as the product increases. By monitoring this, it becomes possible to carry out real time tracking of the nucleic acid amplification reaction.

Since the amplified fragment obtained by the nucleic acid amplification reaction of the invention consists of usual bases, it is also possible to subclone this into an appropriate vector using a restriction enzyme site inside the amplified product. Also, like the case of RFLP, it is also possible to subject the aforementioned amplified fragment to a restriction enzyme treatment, and this be broadly used also in the field of gene inspections. In addition, since the aforementioned amplified fragment can be formed as a product containing the promoter sequence of an RNA polymerase, it becomes possible to synthesize an RNA directly from the amplified fragment. The RNA synthesized in this way can be used as an RNA probe, siRNA and the like.

Also, according to the nucleic acid amplification method of the invention, a base labeled with biotin or a fluorescence material can be used as the substrate, instead of the usual dNTP, which renders possible preparation of a DNA probe labeled with biotin or a fluorescence material. In addition, it is also possible to verify the presence or absence of the amplified product via a certain structure of biotin, a fluorescence material or the like.

It is possible to judge the presence or absence of a mutation in a target nucleic acid sequence in a nucleic acid sample, by making use of the nucleic acid amplification reaction which uses the primer of the invention. For this purpose, the primer of the invention is designed in such a manner that the mutation site to be tested is contained in the primer [preferably a region (a) constituting the primer, more preferably around 3' -end of the region (a)], which renders possible judgment of the presence or absence of mutation by verifying the presence or absence of the amplified product.

Accordingly, the invention provides a method for detecting the presence or absence of a mutation in a target nucleic acid sequence, which comprises the following steps of
(1) carrying out an amplification reaction of the target nucleic acid sequence in a nucleic acid sample, in a reaction system wherein the nucleic acid sample containing the target nucleic acid sequence and the primer of the invention are present, and
(2) judging the presence or absence of a mutation in the target nucleic acid sequence based on the presence or absence of the product of the nucleic acid amplification reaction.

According to the mutation detection method of the invention, when a primer is used which causes a mismatch with the template nucleic acid due to the presence of the mutation of interest, the presence of the amplified product after the nucleic acid amplification reaction indicates the absence of said mutation, and the absence or reduction of the amplified product indicates the presence of said mutation. On the other hand, when an amplified product was obtained using a primer which causes a mismatch with the template nucleic acid due to the absence of the mutation of interest, it can be judged that said mutation is present in the nucleic acid sample, and when the amplified product was not obtained on the contrary, it can be judged that the aforementioned mutation is not present. In this connection, the term "reduction of the amplified product" indicates that the amount of the obtained amplification product is reduced in comparison with the amount of the amplification product obtained when the target nucleic acid sequence is present in the nucleic acid sample.

In addition, it is possible to judge the presence or absence of methylation in a target nucleic acid sequence in a nucleic acid sample, by making use of the nucleic acid amplification reaction which uses the primer of the invention. For this purpose, a treatment is carried out to replace the methylated base in the nucleic acid sample with other base prior to the nucleic acid amplification reaction, and then the nucleic acid amplification reaction is carried out using the primer of the invention designed in such a manner that the site to be tested for methylation is contained in the primer [preferably a region (a) constituting the primer, more preferably around 3'-end of the region (a)]. Thereafter, judgment of the presence or absence of mutation can be made by verifying the presence or absence of the amplified product.

As the treatment for replacing the methylated base with other base, it is not particularly limited and a method well known in said technical field may be optionally used. When a target nucleic acid sample is treated with hydrogen sulfite, un-methylated C (cytosine) is converted into U (uracil), while methylated C is not converted. Thus, the methylated sample can be distinguished from the un-methylated sample when the hydrogen sulfite treatment is carried out and then the amplification reaction is carried out using a set of primers corresponding to the respective bases.

Accordingly, the invention provides a method for detecting the presence or absence of methylation in a target nucleic acid sequence, which comprises the following steps of
(1) carrying out a treatment for replacing a methylated base in a nucleic acid sample containing the target nucleic acid sequence with another base,
(2) carrying out an amplification reaction of the target nucleic acid sequence using the primer of the invention which comprises the site to be tested for methylation, and
(3) judging the presence or absence of methylation in the target nucleic acid sequence based on the presence or absence of the product of the nucleic acid amplification reaction.

According to the methylation detection method of the invention, when a primer is used which causes a mismatch with the template nucleic acid due to the presence of a mutation at the site to be tested for methylation, the presence of the amplified product after the nucleic acid amplification reaction indicates the absence of methylation, and the absence or reduction of the amplified product indicates the presence of said methylation. On the other hand, when an amplified product was obtained using a primer which causes a mismatch with the template nucleic acid due to the absence of a mutation at the site to be tested for methylation, it can be judged that said methylation is present in the nucleic acid sample, and when the amplified product was not obtained on the contrary, it can be judged that said methylation is not present.

Specificity of the mutation detection method and methylation detection method of the invention can be improved by making use of a mutation recognizing protein. Illustratively, when the nucleic acid to be tested contained in a nucleic acid sample comprises a nucleotide different from the target nucleic acid sequence, at the mutation site to be tested or methylation site to be tested, it hybridizes with the target nucleic acid of the primer of the invention to inhibit hybridization of the region (a) which could become an amplification starting point, with the nucleic acid to be tested, so that the amplification product cannot be obtained or amount of the amplification product is reduced.

However, there are cases in which the aforementioned hybridization cannot be prevented perfectly, and in that case, a small amount of a hetero double-stranded structure is formed in these sequences. In this connection, the term "hetero double-stranded structure" means a substantially complementary double-stranded structure but a double-stranded structure which contains a non-complementary region due to the possession of one or two or more mismatches. A wrong amplification product is formed by such a hetero double-stranded structure. In that case, by adding a mutation recognizing protein in advance to the reaction liquid to be used in the nucleic acid amplification reaction, this mutation recognizing protein binds to the aforementioned hetero double-stranded structure, so that the amplification reaction thereafter is prevented. Accordingly, it becomes possible to prevent formation of a wrong amplification product by making use of the mutation recognizing protein.

In order to carry out the nucleic acid amplification method or nucleic acid detection method of the invention, a kit can be prepared by collecting necessary reagents. Accordingly, the kit of the invention comprises the primer of the invention.

In addition, when the primer of the invention comprises a region which can be bonded with a solid phase carrier, it is desirable that the kit of the invention further comprises said solid phase carrier. Also, when the substrate to be used in the nucleic acid amplification reaction comprises a region which can be bonded with a solid phase carrier, it is desirable also that the kit of the invention further comprises said solid phase carrier.

The kit of the invention may further comprise DNA polymerase and the like nucleic acid synthases, dNTP and the like substrates, a buffer, a mutation recognizing protein, a melting temperature adjusting agent and the like aforementioned reagents, a reaction container and specifications. By the use of such a kit, it becomes possible to carry out the nucleic acid amplification reaction by merely adding a template nucleic acid or a nucleic acid sample to the aforementioned reaction container and keeping said reaction container at a certain temperature.

### [Examples]

The following illustratively describes the invention based on examples, but the scope of the invention is not limited to these examples.

### Example 1

### Amplification of target nucleic acid sequence in human β-actin gene

Amplification of a target nucleic acid sequence in a human β-actin gene was carried out using the primer of the invention.

### (1) Preparation,of a nucleic acid sample liquid containing a target nucleic acid fragment

A 100 ng portion of human genomic DNA (mfd. by Clontech) was heated at 98°C for 3 minutes to convert it into single strand, and then amplification of a sequence in the β-actin gene was carried out under the following conditions. A sample was also prepared as a negative control by heating water under the same conditions as described in the above.

### <Primers>

Primers were designed using a human β-actin gene (GenBank accession No: AC006483, target nucleic acid sequence: 171170^{th} to 171282^{nd} nucleotides counting from the 5'-end) as the template. A forward primer <1> (SEQ ID NO:1) was designed in such a manner that it comprised a 3'-end region (the wavy line part of SEQ ID NO:1) which is complementary to the template and a 5' -end region (the underlined part of SEQ ID NO:1) that hybridizes with a region presenting 10 bases downstream from the 3' -end base on the elongation strand of the primer, wherein 4 T bases were arranged between the aforementioned 5'-end region and 3'-end region. A reverse primer <2> (SEQ ID NO:2) consists of a 3'-end region (the wavy line part of SEQ ID NO:2) which is complementary to the template and a 5'-end region which was designed in such a manner that it comprised a sequence (the underlined part of SEQ ID NO:2) complementary to a 3'-end region partial sequence (the bold-faced parts of SEQ ID NO:2), wherein 4 T bases were arranged between the 5'-end region and 3' -end region. In addition, outer primers <3> and <4> (OF and OR) (SEQ ID NOs : 3 and 4) were designed for the outside of each of the forward primer and reverse primer.

**Table 1**

| Forward primer <1>: |
|---|
| 5'-CTCTGGGCCTCGTCGCTTTTGGGCATGGGTCAGAAGGATT-3' (SEQ ID NO:1) |

| Reverse primer <2>: |
|---|
| 5'-ACATGTTTT**CATGT**CGTCCCAGTT**GGTGA**-3' (SEQ ID NO:2) |

| Outer primer <3> (OF): |
|---|
| 5'-GGGCTTCTTGTCCTTTCCTTC-3' (SEQ ID NO:3) |

| Outer primer <4> (OR): |
|---|
| 5'-CCACACGCAGCTCATTGTAG-3' (SEQ ID NO:4) |

### (2) Nucleic acid amplification reaction

An amplification reaction was effected by carrying out the reaction at 60°C for 90 minutes using the following reaction liquid composition.

**<Composition of reaction liquid>**

| | |
|---|---|
| 10 x Bst buffer (DF) | 2.5 µl |
| 100 mM MgSO₄ | 1.5 µl |
| 10% (v/v) Tween 20 | 0.25 µl |
| 100% DMSO | 1.25 µl |
| 25 mM dNTP | 1.4 µl for each |
| SYBR Green I | 0.5 µl |
| 50 µM primer <1> | 1.6 µl |
| 50 µM primer <2> | 1.6 µl |
| 50 µM primer <3> | 0.2 µl |
| 50 µM primer <4> | 0.2 µl |
| Bst. Polymerase | 1.0 µl |
| Taq MutS | 1.0 µl |
| Nucleic acid fragment sample liquid obtained in (1) (100 ng/µl) | 1.0 µl |
| Purified water | 11.0 µl |
| Total volume | 25.0 µl |

### (3) Detection of amplified product

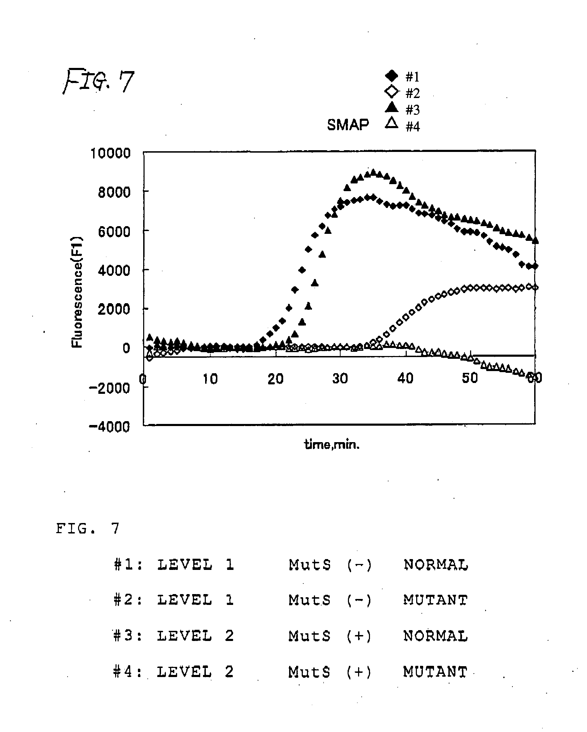

After completion of the amplification reaction of the aforementioned (2), 5 µl of the reaction liquid was applied to a 2% agarose gel (1 x TAE) to carry out electrophoresis, thereby verifying the amplified product. The results are shown in Fig. 6.

As shown in Fig. 6, the amplified product was found only in the nucleic acid-derived sample. In addition, a clear peak was observed at around 120 bp, and since this was predicted from the primer design information, it was found that the aimed amplified product was obtained for certain.

### Example 2

### Detection of one base mutation and effect of MutS

By carrying out a nucleic acid amplification reaction using the primer of the invention in a reaction system in the presence of a mutation recognizing protein (MutS), effect of MutS on the detection of one base mutation was examined.

### (1) Preparation of a nucleic acid sample liquid containing a target nucleic acid fragment

A 100 ng portion of human genomic DNA (mfd. by Clontech) was heated at 98°C for 3 minutes to convert it into single strand, and then amplification of a sequence in the β-actin gene was carried out under the following conditions.

### <Primers>

Primers were designed using a human β-actin gene (GenBank accession No: AC006483, target nucleic acid sequence: 171170^{th} to 171282^{nd} nucleotides counting from the 5'-end) as the template. In order to prepare a model system of one base mutation, primers for mutation detection use (SEQ ID NOs:5 and 6) were newly prepared, and a normal primer <5> (SEQ ID NO:5) having a sequence which matches with the template (the underlined part of SEQ ID NO:5) and a mutant primer <6> (SEQ ID NO: 6) having an artificial mutation at the 3'-end (the underlined part of SEQ ID NO:6) were prepared. As the other primers <1> to <4>, the same primers <1> to <4> used in Example 1 were used.

**Table 2**

| Forward primer <1>: |
|---|
| 5'-CTCTGGGCCTCGTCGCTTTTGGGCATGGGTCAGAAGGATT-3' (SEQ ID NO:1) |

| Reverse primer <2>: |
|---|
| 5'-ACATGTTTT**CATGT**CGTCCCAGTTGGTGA-3' (SEQ ID NO:2) |

| Outer primer <3> (OF): |
|---|
| 5'-GGGCTTCTTGTCCTTTCCTTC-3' (SEQ ID NO:3) |

| Outer primer <4> (OR): |
|---|
| 5'-CCACACGCAGCTCATTGTAG-3' (SEQ ID NO:4) |

| Primer for mutation detection use <5> (wild type) |
|---|
| 5'-CTCTGGGCCTCGTCGC-3' (SEQ ID NO:5) |

| Primer for mutation detection use <6> (mutation type) |
|---|
| 5'-CTCTGGGCCTCGTCGT-3' (SEQ ID NO: 6) |

### (2) Nucleic acid amplification reaction

An amplification reaction was effected by carrying out the reaction at 60°C for 1 hour using the following reaction liquid composition. Level 1 is a level at which MutS was not added, and Level 2 is a level at which MutS was added.

**(Level 1)**

| | |
|---|---|
| 10 x Bst buffer (DF) | 2.5 µl |
| 100 mM MgSO₄ | 1.5 µl |
| 10% (v/v) Tween 20 | 0.25 µl |
| 100% DMSO | 1.25 µl |
| 25 mM dNTP | 1.4 µl for each |
| SYBR Green I | 0.5 µl |
| 50 µM primer <1> | 1.6 µl |
| 50 µM primer <2> | 1.6 µl |
| 50 µM primer <3> | 0.2 µl |
| 50 µM primer <4> | 0.2 µl |
| 50 µM primer <5> or <6> | 0.2 µl |
| Bst. Polymerase | 1.0 µl |
| Nucleic acid fragment sample liquid obtained in (1) (100 ng/µl) | 1.0 µl |
| Purified water | 11.2 µl |
| Total volume (Level 2) | 25.0 µl |
| 10 x Bst buffer (DF) | 2.5 µl |
| 100 mM MgSO₄ | 1.5 µl |
| 10% (v/v) Tween 20 | 0.25 µl |
| 100% DMSO | 1.25 µl |
| 25 mM dNTP | 1.4 µl for each |
| SYBR Green I | 0.5 µl |
| 50 µM primer <1> | 1.6 µl |
| 50 µM primer <2> | 1.6 µl |
| 50 µM primer <3> | 0.2 µl |
| 50 µM primer <4> | 0.2 µl |
| 50 µM primer <5> or <6> | 0.2 µl |
| Bst. Polymerase | 1.0 µl |
| TaqMutS | 1.0 µl |
| Nucleic acid fragment sample liquid obtained in (1) (100 ng/µl) | 1.0 µl |
| Purified water | 10.2 µl |
| Total volume | 25.0 µl |

### (3) Detection of amplified product

The amplification reaction of the aforementioned (2) was carried out by adding SYBR Green I (mfd. by Cambrex) to the reaction liquid to a concentration of 1/100,000 of the initial concentration. Thereafter, fluorescence detection was carried out using a real time fluorescence detector (Mx3000p, mfd. by Stratagene) until 60 minutes after the commencement of the reaction to follow the amplification reaction.

As a result, as shown in Fig. 7, it was found that the amplification occurred from the normal primer within 20 minutes at both of the Levels 1 and 2. In addition, at the Level 2 in which MutS was added, amplification from the mutant primer did not occur in and after 60 minutes of the commencement of the reaction. It can be seen from this that nonspecific amplification is inhibited by MutS.

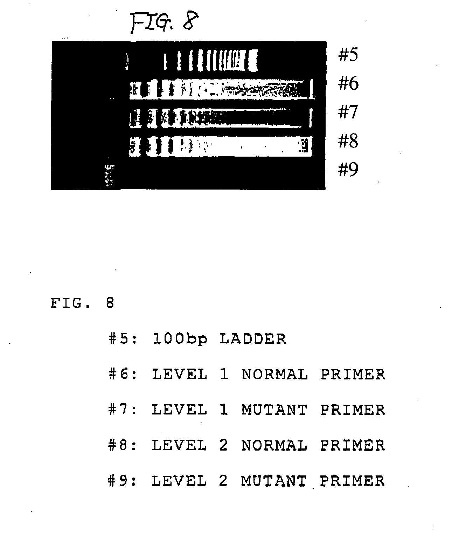

In addition, using 5 µl of the reaction liquid after completion of the amplification reaction of the aforementioned (2), the amplified product was also verified by an electrophoresis using a 2% agarose gel (1 x TAE). As a result, as shown in Fig. 8, it was found that the amplified product is not found when the mutant primer of the Level 2 is used, but the amplified product is obtained in similar amount when the normal primers of the Levels 1 and 2 are used.

The primer of the invention has an advantage of being high in the degree of freedom for primer designing, because, as shown in Fig. 2, the region which hybridizes with the target nucleic acid may have only one region.

The entire disclosure of each and every foreign patent application from which the benefit of foreign priority has been claimed in the present application is incorporated herein by reference, as if fully set forth.

## Claims

1. A primer for amplifying a target nucleic acid sequence, which comprises:
a sequence region (a) complementary to a sequence region (a') in the target nucleic acid sequence; and
a sequence region (b) having a sequence complementary to a partial sequence of the sequence region (a), in this order from a 3' terminal side to a 5' terminal side of the primer.

2. The primer according to claim 1,
wherein each chain length of the sequence regions (a) and (b) is 50 bases or less.

3. The primer according to claim 1,
wherein a chain length of the sequence complementary to the partial sequence of the sequence region (a) is 10 bases or less.

4. The primer according to claim 1, which is utilized in amplifying a target nucleic acid sequence under an isothermal condition.

5. A method for amplifying a nucleic acid, which comprises:
carrying out an amplification reaction of a target nucleic acid sequence in a reaction system in which a nucleic acid sample containing the target nucleic acid sequence and the at least one primer according to claim 1 are present.

6. The nucleic acid amplification method according to claim 5,
wherein a primer having a nucleic acid sequence region (c) complementary to a region (c') in the target nucleic acid sequence is further present in the reaction system, with the proviso that the region (c') is present at a further 3' terminal side than the region (a') in the target nucleic acid sequence.

7. The method according to claim 5,
wherein a mutation recognizing protein is further present in the reaction system.

8. The method according to claim 7,
wherein the mutation recognizing protein is Muts, MSH2 or MHS6, or a mixture of two or more thereof.

9. The method according to claim 5,
wherein a melting temperature adjusting agent is further present in the reaction system.

10. The method according to claim 9,
wherein the melting temperature adjusting agent is dimethyl sulfoxide, betaine, formamide or glycerol, or a mixture of two or more thereof.

11. The method according to claim 5,
wherein the nucleic acid amplification reaction is carried out under an isothermal condition.

12. A method for detecting presence or absence of a mutation in a target nucleic acid sequence, which comprises the following steps of:
(1) carrying out an amplification reaction of a target nucleic acid sequence in a nucleic acid sample, in a reaction system in which the nucleic acid sample containing the target nucleic acid sequence and the at least one primer according to claim 1 are present; and
(2) judging the presence or absence of a mutation in the target nucleic acid sequence based on presence or absence of a product of the nucleic acid amplification reaction.

13. A method for detecting presence or absence of methylation in a target nucleic acid sequence, which comprises the following steps of:
(1) carrying out a treatment for replacing a methylated base in a nucleic acid sample containing the target nucleic acid sequence with another base;
(2) carrying out an amplification reaction of the target nucleic acid sequence using the at least one primer according to claim 1 that comprises a site to be tested for methylation; and
(3) judging the presence or absence of methylation in the target nucleic acid sequence based on presence or absence of a product of the nucleic acid amplification reaction.

14. The method according to claim 13,
wherein the treatment in the step (1) for replacing a methylated base with another base is a treatment with hydrogen sulfite.

15. A kit for nucleic acid amplification, which comprises at least:
the at least one primer according to claim 1;
a nucleic acid synthase;
a substrate; and
a buffer.

16. The kit for nucleic acid amplification according to claim 15, which further comprises a mutation recognizing protein.

17. The kit for nucleic acid amplification according to claim 15, which further comprises a melting temperature adjusting agent.


SEQUENCE LISTING

<110>  Fuji Photo Film Co. Ltd.,

<120>  The primer for gene amplification

<130>  207-02547

<150>  JP2006-183299
<151>  2006-07-03

<160>  6

<170>  PatentIn version 3.1

<210>  1
<211>  40
<212>  DNA
<213>  Artificial

<220>
<223>  Artificially synthesized oligonucleotide sequence

<400>  1
ctctgggcct cgtcgctttt gggcatgggt cagaaggatt                                40


<210>  2
<211>  29
<212>  DNA
<213>  Artificial

<220>
<223>  Artificially synthesized oligonucleotide sequence

<400>  2
acatgttttc atgtcgtccc agttggtga                                            29


<210>  3
<211>  21
<212>  DNA
<213>  Artificial

<220>
<223>  Artificially synthesized oligonucleotide sequence

<400> 3
gggcttcttg tcctttcctt c 21

<210> 4
<211> 20
<212> DNA
<213> Artificial

<220>
<223> Artificially synthesized oligonucleotide sequence

<400> 4
ccacacgcag ctcattgtag 20

<210> 5
<211> 16
<212> DNA
<213> Artificial

<220>
<223> Artificially synthesized oligonucleotide sequence

<400> 5
ctctgggcct cgtcgc 16

<210> 6
<211> 16
<212> DNA
<213> Artificial

<220>
<223> Artificially synthesized oligonucleotide sequence

<400> 6
ctctgggcct cgtcgt 16